# EUROPÄISCHE PATENTANMELDUNG

(11) **EP 1 348 912 A1**
(43) Veröffentlichungstag der Anmeldung: **01.10.2003**
(21) Anmeldenummer: 02026308.3
(22) Anmeldetag: 20.11.2002
(51) Int. Cl.: F24F 3/16

(54) **Luftverbesserer und Verfahren zur Luftverbesserung in Räumen**

(30) Priorität: 27.03.2002 DE 10213889; 17.06.2002 US 173140
(71) Anmelder: Weigl, Lidia, Englewood, Florida 34223 (US)
(72) Erfinder: Weigl, Adolf, Englewood, Florida 34223 (US)
(74) Vertreter: Lange, Thomas, Dr.

(57) **Zusammenfassung**

Eine Vorrichtung zur Luftverbesserung für ein Klimatisierungssystem umfasst eine Dosiereinrichtung (2, 11, 13), welche eine steuerbare Dosierung der pro Zeit abgegebenen Menge eines Wirkstoffes vornimmt. Ferner umfasst sie einen saugfähigen Aufnahmeträger (5), auf welchen der von der Dosiereinrichtung (2, 11, 13) abgegebene Wirkstoff aufdosiert wird.

## Beschreibung

Die Erfindung betrifft eine Vorrichtung sowie ein Verfahren zur Luftverbesserung in klimatisierten Räumen.

Praktisch in sämtlichen von Menschen genutzten Räumen können unangenehme oder störende Gerüche auftreten, welche das Wohlbefinden von darin verweilenden Personen beeinträchtigen. In klimatisierten Räumen wie beispielsweise Supermärkten, Banken, Haarsalons, Restaurants, Diskotheken, aber auch in einem Wohnhaus oder dem Innenraum des Autos, werden störende Gerüche in vielen Fällen durch den Eigengeruch der laufenden Klimaanlage hervorgerufen. Weitere Geruchsemmissionen können durch Tätigkeiten wie Kochen usw. hervorgerufen werden oder, insbesondere bei großen Menschenmengen in geschlossenen Räumen, durch menschliche Ausdünstungen bewirkt werden.

Es sind verschiedene Maßnahmen bekannt, um Missgerüche in Räumen zu neutralisieren bzw. zu übertönen. Für das Auto werden sogenannte Dufttannenbäume angeboten, welche, hinter den Rückspiegel gehängt, den Geruch im Auto verbessern sollen. Diese Dufttannenbäume, bei denen es sich um mit einem Aromastoff getränkte Fleece handelt, wirken jedoch nur kurze Zeit.

Ein weiteres bekanntes Produkt im Bereich der Massenartikel ist ein parfümierter Filzring, welcher von oben auf die Glühlampe gelegt wird. Wenn die Glühlampe eingeschaltet wird und somit Wärme produziert, gibt der Filzring vermehrt Duftstoffe ab. Auch dieses Produkt weist eine sehr begrenzte Lebensdauer sowie den Nachteil auf, dass die Geruchsemmission anfangs zu stark und später zu schwach, d.h. nicht dosierbar ist.

Ferner sind insbesondere für den Gebrauch in Privathäusern elektrisch betriebene Duftspender bekannt, welche in die Steckdose eingesteckt werden. Durch den aus der Steckdose bezogenen Strom wird über eine Heizwicklung Wärme produziert, welche parfümierte Flüssigkeiten, Gele oder Kristalle verdampfen lässt. Auch diese Duftspender verbrauchen sich sehr schnell, wirken nur lokal und können nicht zentral gesteuert werden.

Ferner ist es bereits bekannt, Duftspender mit einer Klimaanlage zu koppeln. In der U.S.-Patentschrift 6,264,887 B1 ist eine Klammer aus einem parfümierten Material beschrieben, welche in das Austrittsgitter einer Klimaanlage gesteckt wird.

Die Schrift U.S. 6,270,720 B1 zeigt eine Klimaanlage, in welcher eine mit einer verdampfbaren Flüssigkeit befüllbare Rinne untergebracht ist. Durch eine Heizung der Rinne kann ein darin befindlicher Duftstoff verdampft werden. Nachteilig ist, dass auch bei deaktivierter Heizung ein Verbrauch von Duftstoff auftritt, zumindest solange, bis sämtliche Flüssigkeit aus der Rinne verdampft ist.

Die Schrift U.S. 6,065,301 betrifft ein System zur Reinigung eines Verdampfers in einer Kfz-Klimaanlage oder zum Zuführen von geruchsaktiven oder antibakteriellen Stoffen in die Anlage. Hierfür ist ein im Strömungsweg hinter dem Gebläse angeordneter Düsenstab zur Zersteubung der entsprechenden Flüssigkeit vorgesehen. Eine kontinuierliche Anreicherung des Luftstroms mit den aromatischen oder antibakteriellen Wirkstoffen ist nicht möglich.

In den Schriften U.S. 5,963,302 und U.S. 5,832,320 sind Anlagen beschrieben, mit welchen verschiedene Geruchsstoffe über ein Revolver ähnliches Duftrad einer Belüftungsanlage zugesetzt werden können. Die Dosierungssteuerung erfolgt über eine zuschaltbare Bypass-Leitung. Gedacht ist an eine Verwendung in Kinos und dergleichen.

Die Schrift U.S. 5,957,771 betrifft einen in eine Klimaanlage integrierten Aroma-Spraymechanismus, welcher mittels eines Magnetventils realisiert ist. Der Spraymechanismus ermöglicht lediglich eine stoßweise Anreicherung des Luftstroms in der Klimaanlage mit Duftstoffen.

Der Erfindung liegt die Aufgabe zugrunde, eine Vorrichtung sowie ein Verfahren zur Luftverbesserung in Räumen zu schaffen, welches eine kontinuierliche Anreicherung eines Luftstroms mit einem Wirkstoff ermöglicht. Insbesondere soll die Vorrichtung bzw. das Verfahren in Klimatisierungssystemen für unterschiedlichste Raumgrößen einsetzbar sein und bei unterschiedlichsten Bedingungen den in der Praxis gestellten Anforderungen genügen. Ferner soll insbesondere auch eine gute Dosierbarkeit erreichbar sein.

Die Erfindung wird durch die Merkmale der unabhängigen Ansprüche 1 und 23 gelöst. Vorteilhafte Weiterbildungen der Erfindung sind Gegenstand der Unteransprüche.

Gemäß Anspruch 1 umfasst die erfindungsgemäße Vorrichtung eine Dosiereinrichtung, welche eine steuerbare Dosierung der pro Zeit abgegebenen Menge eines Wirkstoffes vornimmt. Ferner weist die Vorrichtung einen in einem Kanal des Klimatisierungssystems vorgesehenen saugfähigen Aufnahmeträger auf, auf welchen der von der Dosiereinrichtung abgegebene Wirkstoff aufdosiert wird.

Der saugfähige Aufnahmeträger bewirkt, dass der von der Dosiereinrichtung abgegebene Wirkstoff nicht schlagartig sondern über eine bestimmte Zeitdauer dem Luftstrom in dem Klimatisierungssystem zugesetzt wird. Durch die Dosiereinrichtung kann ein gewünschter Durchtränkungsgrad des Aufnahmeträgers eingestellt und aufrechterhalten werden. Dadurch ist eine kontinuierliche Anreicherung des Luftstroms mit dem Wirkstoff zu erreichen. Gleichwohl weist die Vorrichtung aufgrund der Dosiereinrichtung eine gute und über einen weiten Bereich steuerbare Variabilität der Wirkstoffzumischung auf, so dass unterschiedlichsten Bedingungen (kleine Räume oder großvolumige Gebäude, Betrieb des Klimatisierungssystems im Kühloder Heizbetrieb) Rechnung getragen werden kann.

Vorzugsweise steht die Dosiereinrichtung mit einer Steuereinheit einer Klimaanlage des Klimatisierungssystems in Verbindung. In der einfachsten Form besteht die Steuerung darin, dass die Dosiereinrichtung entsprechend dem Ein/Aus-Betriebszustand der Klimaanlage aktiviert bzw. deaktiviert wird, z.B. indem sie von der geschalteten Betriebsspannung der Klimaanlage versorgt wird. Dadurch wird erreicht, dass die Dosiereinrichtung nur dann in Betrieb ist und den Wirkstoff abgibt, wenn die Klimaanlage läuft. Andererseits wird durch das Abschalten der Klimaanlage auch die Dosiereinrichtung abgeschaltet. Folglich wird verhindert, dass bei abgeschalteter Klimaanlage (z.B. bei Geschäftsräumen am Wochenende) ein Wirkstoffverbrauch auftritt.

Ferner kann auch die Dosiermenge in Abhängigkeit von dem Betriebszustand der Klimaanlage gesteuert werden. Bei eingeschalteter Klimaanlage ist z.B. eine Steuerung der Dosiereinrichtung in Abhängigkeit von dem Luftdurchsatz in der Klimaanlage (d.h. der Ventilatorleistung in der Klimaanlage) oder von anderen Betriebsparametern wie beispielsweise einer Kühlung oder Erwärmung der Luft in der Klimaanlage möglich. Es kann somit für praktisch alle Situationen und Bedingungen eine genaue Abstimmung der Dosiereinrichtung auf die verwendete Klimaanlage erreicht werden.

Bei der Dosiereinrichtung handelt es sich vorzugsweise um eine elektromechanische Dosiereinrichtung, wenngleich auch rein mechanisch arbeitende Dosiereinrichtungen, z.B. solche wie man sie aus medizinischen Anwendungen kennt (Tropf-Infusionen, bei welchen ein den Wirkstoff leitender flexibler Schlauch durch ein asymmetrisches Nockenrad oder eine andere Quetschmechanik in einstellbarer Weise zusammengepreßt wird), möglich sind. Bei rein mechanischen wirkenden Dosiereinrichtungen wird die Dosierungseinstellung manuell durchgeführt, eine Ein/Aus-Steuerung kann jedoch auch hier über ein von der Klimaanlage gesteuertes elektrisches Absperrventil vorgesehen sein.

Eine elektromechanische Dosiereinrichtung verwendet vorzugsweise einen Elektromotor, insbesondere einen Synchron-Elektromotor, als elektromechanischen Antrieb. Die Dosierung wird in diesem Fall durch die Steuerung der Drehzahl des Elektromotors beeinflusst.

Nach einer besonders bevorzugten Ausgestaltung der Dosiereinrichtung umfasst diese ein erstes Teil, welches über eine Zuleitung mit einem Wirkstoffreservoir verbunden ist, und welches eine Öffnung zur Abgabe des Wirkstoffes aufweist, ein zweites Teil, welches eine Transportvertiefung zur Aufnahme des aus der Öffnung des ersten Teils abgegebenen Wirkstoffs umfasst, und einen elektromechanischen Antrieb, der eine Relativbewegung der beiden Teile herbeiführt, derart, dass die Transportvertiefung an der Öffnung zur Abgabe des Wirkstoffes vorbeigeführt und dabei befüllt wird. Das Aufnahmevolumen der Transportvertiefung definiert dabei die kleinste zu dosierende Menge des Wirkstoffes.

Vorzugsweise ist das erste Teil ein Zylinder, dessen Innenwandung mit der Öffnung zur Abgabe des Wirkstoffes versehen ist, und das zweite Teil ist eine Rotationswalze, deren Mantelfläche mit der Transportvertiefung versehen ist. Eine solche elektromechanische Dosiereinrichtung ist mechanisch robust und für den Langzeiteinsatz auch in großvolumigen Klimatisierungssystemen geeignet.

Dadurch, dass über den Umfang der Mantelfläche verteilt mehrere Transportvertiefungen vorgesehen sind, wird die Transportkapazität der Dosiereinrichtung durch Verkleinerung der Dosierzeitintervalle bei gegebener Drehzahl der Rotationswalze erhöht.

Eine weitere vorteilhafte Ausgestaltung der Erfindung kennzeichnet sich dadurch, dass das erste Teil mehrere Öffnungen zur Abgabe des Wirkstoffes und das zweite Teil mehrere Transportvertiefungen zur Entgegennahme des Wirkstoffes aufweisen. Durch selektives Verschließen von Auslassöffnungen, die mit den Öffnungen zur Abgabe des Wirkstoffes und den Transportvertiefungen korrespondieren, kann die Vorrichtung an unterschiedlichste Dimensionierungen des Klimatisierungssystems bzw. der darin enthaltenen Klimaanlage angepasst bzw. für unterschiedliche Geruchsstärken eingesetzt werden. Darüber hinaus ermöglichen eine Mehrzahl von Auslassöffnungen eine gleichmäßigere und großflächigere Verteilung des Wirkstoffes über den saugfähigen Aufnahmeträger, was ebenfalls die Kapazität und das Betriebsverhalten der Vorrichtung günstig beeinflusst.

Vorzugsweise besteht der saugfähige Aufnahmeträger aus einem faserhaltigen und/oder offenporigen Material, insbesondere Zellstoff oder einem Saugpapier. Diese Materialien ermöglichen einerseits eine gute laterale Verteilung des Wirkstoffes in dem Aufnahmeträger und schaffen damit eine Verdampfungsfläche ausreichender Größe, und zeigen andererseits ein Speicherverhalten für den Wirkstoff, welches bewirkt, dass auch bei einer zeitdiskreten Wirkstoff-Zudosierung eine über die Zeit im wesentlichen gleichmäßige Abgabe des Wirkstoffes an den Luftstrom erreicht wird.

Vorzugsweise umfasst die Vorrichtung eine Halterung für den Aufnahmeträger, welche baulich fest mit der Dosiereinrichtung verbunden ist. Die Vorrichtung bildet in diesem Fall eine bauliche Einheit, welche einfach in den dafür vorgesehenen Kanal des Klimatisierungssystems zu montieren ist.

Vorzugsweise ist die Halterung als ein Wechselhalter, insbesondere in Form eines Einschubrahmens, für den Aufnahmeträger ausgebildet. Durch diese Maßnahme wird ein einfaches Austauschen von verschmutzten oder aus anderen Gründen zu erneuernden Aufnahmeträgern ermöglicht.

Bei dem Wirkstoff handelt es sich vorzugsweise um eine Duftessenz und/oder eine antibakteriell wirkende Essenz. Es können jedoch auch Wirkstoffe mit anderen Wirkungen verabreicht werden.

Vorzugsweise wird die Vorrichtung in einen Kanal eines Klimatisierungssystems zur Klimatisierung eines Raums oder eines Gebäudes eingebaut. Es ist jedoch auch die Verwendung der Vorrichtung in einem Klimatisierungssystem eines Kfz möglich und vorgesehen.

Die Erfindung wird nachfolgend anhand eines Ausführungsbeispieles und Varianten desselben unter Bezugnahme auf die Zeichnung erläutert; in dieser zeigt:
Fig. 1 eine teilweise aufgeschnittene Darstellung einer in einen Kanal eingebauten Vorrichtung nach einem Ausführungsbeispiel der Erfindung in Seitenansicht;
Fig. 2 eine Seitenansicht der in Fig. 1 gezeigten Vorrichtung;
Fig. 3 einen Querschnitt durch den Zylinder entlang der Linie A-A in Fig. 2;
Fig. 4 eine Seitenansicht einer Rotationswalze mit Transportvertiefungen der Vorrichtung;
Fig. 5 einen Querschnitt entlang der Linie B-B in Fig. 4;
Fig. 6 eine perspektivische Darstellung einer Vorrichtung gemäß dem ersten Ausführungsbeispiel der Erfindung;
Fig. 7 einen Querschnitt durch eine Rotationswalze mit Gehäuse nach einer Variante des Ausführungsbeispiels; und
Fig. 8 einen Längsschnitt durch die in Fig. 7 gezeigte Rotationswalze mit Gehäuse.

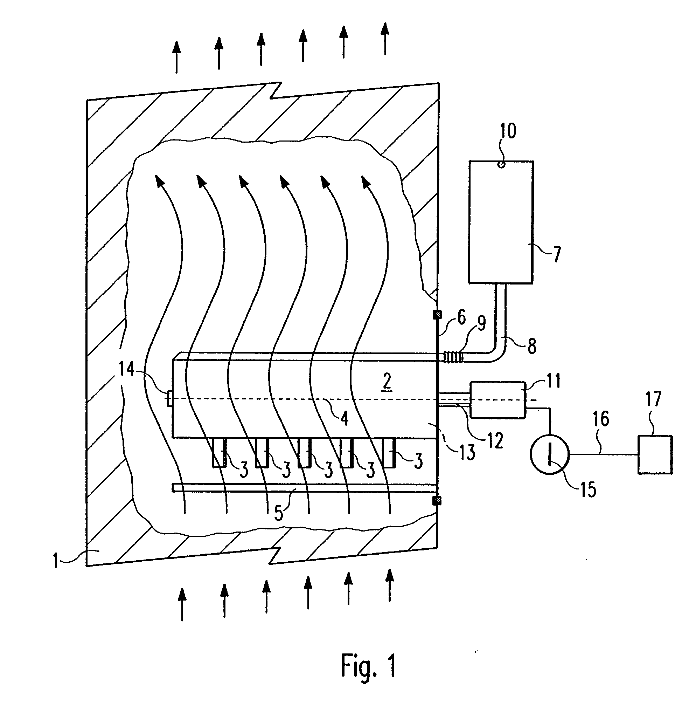

Fig. 1 zeigt in schematischer Darstellung ein Ausführungsbeispiel einer erfindungsgemäßen Luftanreicherungsvorrichtung im Einbauzustand. Ein Kanal 1 eines Klimatisierungssystems erstreckt sich in Fig. 1 in senkrechter Richtung und ist teilweise aufgeschnitten dargestellt. Der Luftstrom vor, durch und hinter dem gezeigten Kanalabschnitt ist durch Pfeile angedeutet.

Klimatisierungssysteme für Gebäude oder Häuser sind aus Gründen der Energieeinsparung als Kreislaufsysteme konzipiert. Luft wird entweder in Bodennähe oder durch Schächte im Dekkenbereich aus einem Raum oder mehreren Räumen abgezogen und über ein Kanalsystem einer zentralen Klimaanlage (nicht dargestellt) zugeleitet. Die Klimaanlage umfasst ein Kaltluftaggregat (Verdampfer), meistens auch ein Heizsystem und ein oder mehrere Flügelräder zum Transport der die Klimaanlage durchströmenden Luft. Der von der Klimaanlage abgegebene Luftstrom gelangt zunächst in einen Zentralkanal, welcher sich im weiteren Strömungsweg in ein Kanalsystem bestehend aus mehreren Einzelkanälen aufteilt. Das Kanalsystem ist je nach den baulichen Gegebenheiten des zu klimatisierenden Hauses bzw. Gebäudes ausgeführt und so ausgelegt, dass eine wirksame Klimatisierung im gesamten Raum bzw. Gebäude erreicht wird.

Der in Fig. 1 dargestellte Kanalabschnitt 1 befindet sich vorzugsweise im Zentralkanal abstromseitig der Klimaanlage. In diesem Fall wird eine Luftverbesserung in allen von der Klimaanlage versorgten Räumen erreicht. Grundsätzlich kann es sich bei dem Abschnitt 1 jedoch auch um einen bezüglich der Klimaanlage zustromseitig angeordneten Kanal, um einen Kanal innerhalb der Klimaanlage oder um einen Einzelkanal des beschriebenen Kanalsystems handeln. Ferner besteht natürlich auch die Möglichkeit, dass lediglich ein einziger Belüftungskanal vorgesehen ist, d.h. das verzweigte Kanalsystem entfällt.

Bei dem Kanal 1 kann es sich um einen handelsüblichen Kanal für eine Klimaanlage z.B. für ein Wohnhaus handeln. Ein solcher Kanal ist z.B. 50 cm breit, 40 cm tief und besteht aus verzinktem Blech.

Die Luftanreicherungsvorrichtung weist einen in Seitenansicht dargestellten Zylinder 2 auf, welcher an seinem unteren Bereich über die Länge verteilt 5 Auslassöffnungen 3 umfasst. Die Zylinderachse ist durch die gestrichelte Linie 4 dargestellt. Gegenüberliegend der Auslassöffnungen 3 ist ein Aufnahmeträger 5 angeordnet. Der Aufnahmeträger 5 besteht aus einem saugfähigen Material, beispielsweise einem Fleece insbesondere aus neutralem Zellstoff oder Saugpapier. Wie in Fig. 1 ersichtlich, befindet sich der Aufnahmeträger 5 im Luftstrom, so dass ein durch die Auslassöffnungen 3 aufdosierte Wirkstoff im Luftstrom verdunstet und abtransportiert wird.

Der Zylinder 2 sowie der Aufnahmeträger 5 können in später noch näher dargestellter Weise konstruktiv miteinander verbunden und beispielsweise an einem abnehmbaren Element 6 der Kanalwandung montiert sein.

Außerhalb des Kanals 1 befindet sich ein Wirkstoffbehälter 7, welcher über eine Zuleitung 8 mit einem Anschluss 9 oder integrierten Kanal des Zylinders 2 in Flüssigkeitsverbindung steht. Der Wirkstoffbehälter 7 ist in seinem oberen Bereich mit einem Belüftungsventil 10 ausgestattet, welches das Auftreten eines Unterdrucks beim Entleeren des Wirkstoffbehälters 7 vermeidet.

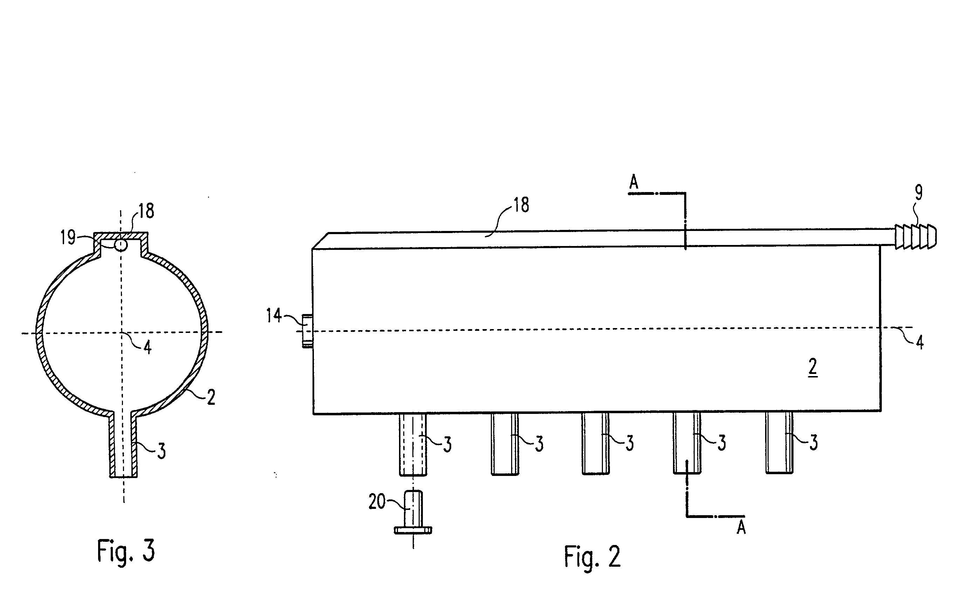

Ein Synchronmotor 11 treibt die Welle 12 einer in Fig. 1 nicht sichtbaren Rotationswalze 13 an, die sich innerhalb des Zylinders 2 befindet und mittels einer Achsaufnahme 14 des Zylinders 2 drehbar in diesem gelagert ist, siehe auch Fig. 2.
Dem Synchronmotor 11 ist ein Drehzahlregler 15 vorgeschaltet, mittels welchem die Drehzahl des Synchronmotors 11 mit hoher Genauigkeit einstellbar ist. Der Drehzahlregler 15 bezieht seine Betriebsspannung über eine elektrische Zuleitung 16 von der in Fig. 1 lediglich schematisch dargestellten Klimaanlage 17. Dadurch wird erreicht, dass bei einem Ausschalten der Klimaanlage 17 automatisch auch der Synchronmotor 11 abgeschaltet wird. Ferner können in nicht dargestellter Weise zwischen einer Steuereinheit der Klimaanlage 17 und dem Drehzahlregler 15 weitere elektrische Steuerleitungen vorgesehen sein, über welche eine über das Ein- und Auschalten hinausgehende Steuerfunktionalität des Drehzahlreglers 15 durch die Klimaanlage 17 ermöglicht wird. Bei einem reduzierten Betrieb der Klimaanlage 17 mit verminderter Ventilatorleistung und somit vermindertem Luftdurchsatz kann proportional dazu auch die Drehzahl des Synchronmotors 11 vermindert werden. Darüber hinaus kann die Steuerung der Motordrehzahl in Abhängigkeit von der Temperatureinstellung der Klimaanlage 17 erfolgen. Derartige Steuerverbindungen können beispielsweise über Datenleitungen realisiert sein, welche an eine programmierbare Schnittstelle der Klimaanlage 17 anzuschließen sind.

Fig. 2 zeigt den Zylinder 2 in vergrößerter Darstellung. Ein Querschnitt entlang der Linie A-A durch den Zylinder 2 ist in Fig. 3 dargestellt. In ihrem oberen Bereich ist die Wandung des Zylinders 2 in Form eines Kanals 18 ausgebildet. In dem parallel zu der Zylinderachse 4 verlaufenden Wandkanal 18 befindet sich ein Zuflussrohr 19, welches mit dem Anschluss 9 in Verbindung steht.

Die Auslassöffnungen 3 können mit Pfropfen oder Verschlusskappen 20 dicht verschlossen werden.

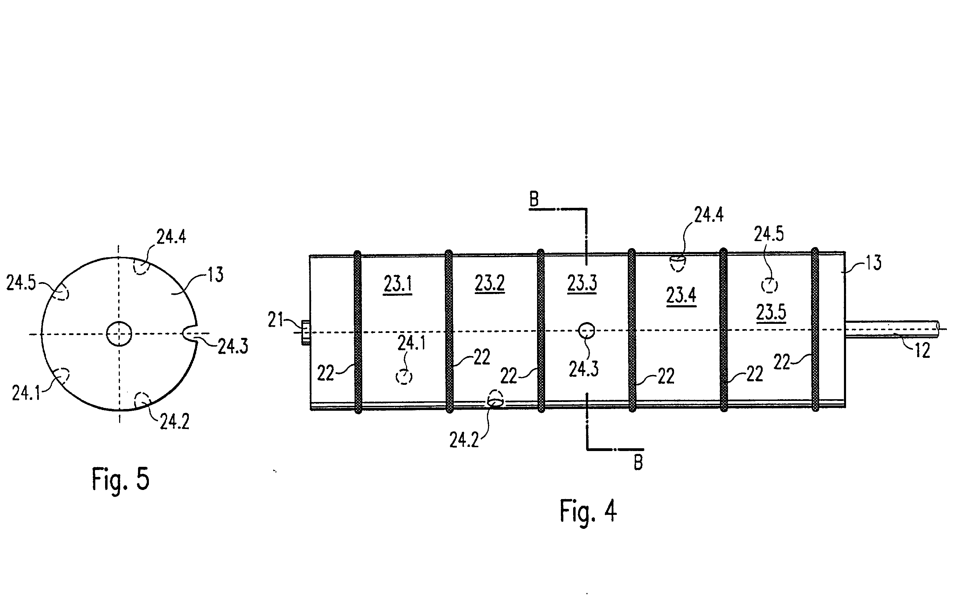

Die Fig. 4 und 5 zeigen die Rotationswalze 13 in einer Seitenansicht bzw. im Querschnitt entlang der Linie B-B. Der Auβendurchmesser der Rotationswalze 13 entspricht bis auf einen minimalen Bewegungsspielraum dem Innendurchmesser des Zylinders 2. Ein zentral an der freien Stirnwand angeordneter Zapfen 21 ist im eingebauten Zustand in der Achsaufnahme 14 des Zylinders 2 gelagert. An der gegenüberliegenden Seite der Rotationswalze 13 erstreckt sich die Welle 12.

Die Rotationswalze 13 wird in dem hier dargestellten Beispiel mittels 6 in gleichen Abständen über die Walzenlänge verteilt angeordneten Dichtringen 22 in 5 gegeneinander abgedichtete Abschnitte 23.1, 23.2, 23.3, 23.4 und 23.5 unterteilt. Die aus einem säure- und ölbeständigen Material, z.B. Neopren, bestehenden Axialdichtringe 22 verhindern einen Flüssigkeitsaustausch zwischen den genannten Abschnitten 23.1 bis 23.5. In jedem Abschnitt 23.1, 23.2, 23.3, 23.4 und 23.5 ist eine Transportvertiefung 24.1, 24.2, 24.3, 24.4 bzw. 24.5 ausgebildet. Fig. 5 zeigt, dass die Transportvertiefungen 24.1 bis 24.5 in Umfangsrichtung äquidistant über die Oberfläche der Rotationswalze 13 verteilt angeordnet sind. Der Radialwinkel zwischen benachbarten Transportvertiefungen beträgt in dem hier gezeigten Beispiel mit 5 Transportvertiefungen 72°. In ihrer axialen Lage korrespondieren die Transportvertiefungen 24.1 bis 24.5 mit den Auslassöffnungen 3 des Zylinders 2.

Die Wirkungsweise der Vorrichtung ist wie folgt. Gelangt eine Transportvertiefung 24.1 bis 24.5 durch Drehung der Rotationswalze 13 in den Bereich des Wandkanals 18, wird sie mit dem Wirkstoff, üblicherweise einer Flüssigkeit, beladen. Die zu transportierende Menge des Wirkstoffes wird durch das Aufnahmevolumen der Transportvertiefung 24.1 bis 24.5 bestimmt und kann z.B. etwa 1 bis 2 g bei Verwendung eines Öls als Wirkstoff-Flüssigkeit betragen. Bei einer Weiterdrehung der Rotationswalze 13 wird die mit dem Wirkstoff gefüllte Transportvertiefung 24.1, 24.2, 24.3. 24.4 bzw. 24.5 an der Innenwandung des Zylinders 2 bis zu der korrespondierenden Auslassöffnung 3 geführt. Durch die praktisch identischen Außenund Innendurchmesser der Rotationswalze 13 und des Zylinders 2 wird gewährleistet, dass der Wirkstoff kontrolliert und ohne Verlust zu der Auslassöffnung 3 gebracht wird. Sobald die Transportvertiefung 24.1 bis 24.5 in den Bereich der Auslassöffnung 3 gelangt, entleert sich die Transportvertiefung 24.1 bis 24.5 und der Wirkstoff gelangt (sofern die Auslassöffnung 3 nicht verschlossen ist) auf den Aufnahmeträger 5.

In der Regel wird durch die Wirkstoff-Flüssigkeit die Schmierung des Systems bewirkt. Um zu verhindern, dass die Rotationswalze 13 in dem Zylinder 2 trocken läuft, wenn der Wirkstoffbehälter 7 leer ist, kann ein Sicherheitsschalter (nicht dargestellt) vorgesehen sein, der bei leerem Wirkstoffbehälter 7 ausgelöst wird und bewirkt, dass die Stromzufuhr über die elektrische Zuleitung 16 zu dem Synchronmotor 11 unterbrochen wird.

Als Sicherheitsschalter kann beispielsweise ein Quecksilberschalter oder ein berührungsfreier Näherungsschalter verwendet werden, der in einem eigenen Messgehäuse (nicht dargestellt) untergebracht ist, welches in die Wirkstoff-Zuleitung 8 integriert ist. Sobald der Flüssigkeitsstand in dem Messgehäuse absinkt, wird dies durch einen Schwimmer (beim Näherungsschalter z.B. ein in säurefestem PU-Schaum eingeschäumter Magnet) registriert und der Schalter bei Unterschreiten eines bestimmten Flüssigkeitsniveaus bzw. bei leerem Messgehäuse ausgelöst. Der Schalter kann dabei auch eine optische Warnanzeige aktivieren.

Im Folgenden wird ohne eine Beschränkung der Allgemeinheit als Wirkstoff-Flüssigkeit ein Duftöl betrachtet. Ein aus Zellstoff bestehender Aufnahmeträger 5 quillt durch die Benetzung mit dem Duftöl etwas auf. Die Saugfähigkeit des Zellstoffträgers bewirkt sowohl eine Verteilung des Duftöls über den Aufnahmeträger 5 als auch eine gewisse Speicherung des Duftöls innerhalb des Aufnahmeträgers 5. Letzteres bewirkt, dass der Eigengeruch des Öls nicht schlagartig sondern über einen längeren Zeitraum an die Umgebung in dem Kanal 1 abgegeben wird.

Versuche in einem etwa 300 qm großen und 3,3 m hohen, komplett klimatisierten Wohnhaus ergaben, dass bei Verwendung eines Filzträgers der Größe 80 x 30 x 2 mm der mit einigen Tropfen eines kommerziell erhältlichen Duftöls benetzte Filzträger nach Einschalten der Klimaanlage in dem gesamten Wohnhaus augenblicklich einen wohlriechenden Duft verbreitete. Ohne eine Wiederbenetzung des Filzträgers hielt dieser Zustand bei einer mittleren Leistung der Klimaanlage etwa 6 bis 7 Stunden an. Danach nahm die Intensität des Duftes langsam ab. Sobald alle 5 bis 6 Stunden zwei bis drei Tropfen des Öls auf den Filzträger aufgebracht wurden, herrschte in allen Räumen des Wohnhauses ein stets gleichbleibender Duft, ohne dass sich die Dosierintervalle bemerkbar machten. Eine entsprechende Auslegung ist für die erfindungsgemäße Vorrichtung vorgesehen.

Die erfindungsgemäße Vorrichtung weist eine Vielzahl von Variationsmöglichkeiten zur Steuerung der Geruchsstärke und zur Anpassung der Anlage an unterschiedliche Einsatzsituationen und Gebäude- oder Raumgrößen auf.

Eine erste Variationsmöglichkeit ergibt sich durch die Verschließbarkeit der Auslassöffnungen 3. Führt der Synchronmotor 11 beispielsweise eine Umdrehung in 24 Stunden aus, können innerhalb von 24 Stunden je nach Anzahl der geöffneten Auslassöffnungen 3 ein bis fünf Tropfen Duftöl auf den Aufnahmeträger 5 aufgebracht werden.

Eine zweite, kontinuierliche Regelmöglichkeit besteht in der Erhöhung der Motordrehzahl.

Konstruktiv kann gemäß einer dritten Variationsmöglichkeit vorgesehen sein, in jedem Abschnitt 23.1 bis 23.5 mehrere über den Umfang verteilt angeordnete Transportvertiefungen 24.1 bis 24.5 vorzusehen. In diesem Fall erhöht sich die Kapazität des Systems.

Eine weitere Variante besteht darin, die Aufnahmevolumen von in verschiedenen Abschnitten 23.1 bis 23.5 liegenden Transportvertiefungen 24.1, 24.2, 24.3, 24.4 und 24.5 unterschiedlich zu gestalten. Durch diese Maßnahme kann der Arbeitsbereich der Vorrichtung durch selektives Verschließen oder Öffnen geeigneter Auslassöffnungen 3 erheblich vergrößert werden.

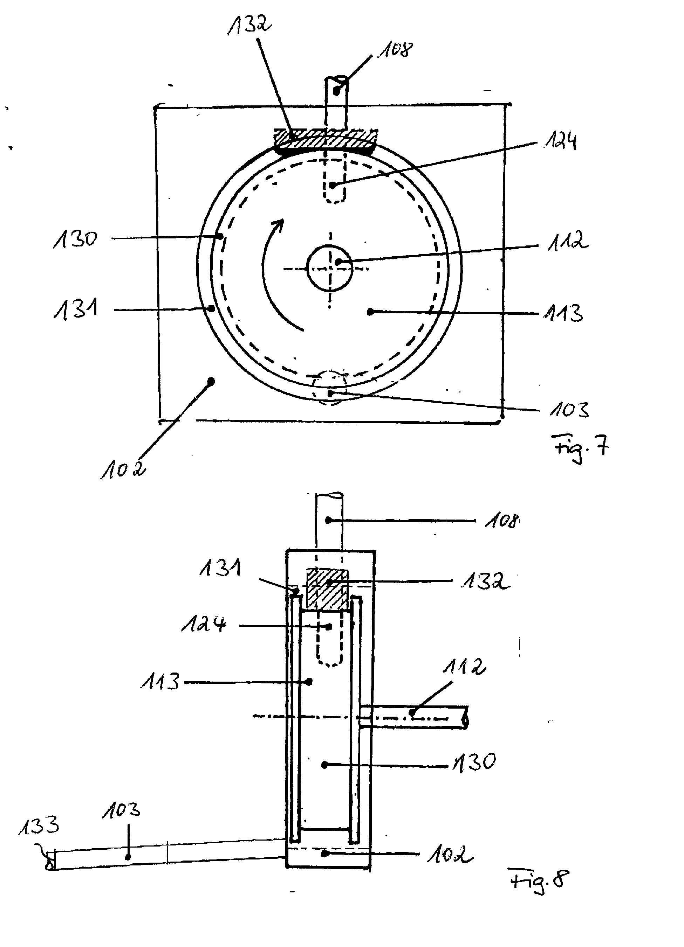

Ferner besteht die Möglichkeit, anstelle der fünf Abschnitte 23.1 bis 23.5 mit jeweils einer Transportvertiefung einen einzelnen Abschnitt (in Form eines Transportrades) mit z.B. fünf über den Umfang verteilt angeordneten Transportvertiefungen vorzusehen. Diese Variante wird noch anhand der Fig. 7 und 8 näher erläutert.

Schließlich besteht die Möglichkeit, mehrere Zuflussrohre 19 vorzusehen und dadurch die gleichzeitige Zugabe von mehreren unterschiedlichen Wirkstoffen zu dem Luftstrom zu ermögliche. Beispielsweise kann neben einem Duftstoff auch ein in einem separaten Behälter (nicht dargestellt) enthaltener antibakterieller Wirkstoff eingesetzt werden. In diesem Fall sind einige der Abschnitte 23.1 bis 23.5 für die Aufdosierung des Duftstoffes und die restlichen Abschnitte 23.1 bis 23.5 für die Aufdosierung des antibakteriellen Wirkstoffes vorgesehen. Der antibakterielle Wirkstoff kann auch anstelle des Duftstoffes eingesetzt werden.

Ferner kann durch Verwendung einer in den Kanal 1 eingebauten UV-Lampe eine (geruchsneutrale) antibakterielle Wirkung erzielt werden. Durch eine intensive Ausleuchtung des Kanals lässt sich eine hohe Effizienz erreichen. Die UV-Lampe kann über die elektrische Zuleitung 16 mit Strom versorgt werden und ist in diesem Fall so geschaltet, dass sie nur dann leuchtet, wenn die Klimaanlage und/oder die erfindungsgemäße Luftanreicherungsvorrichtung in Betrieb sind. Eine andere Möglichkeit besteht darin, die UV-Lampe über einen unabhängigen Stromkreis zu versorgen. Die UV-Lampe leuchtet dann kontinuierlich, wodurch sich ihre Lebensdauer erhöht und auch im ausgeschalteten Betriebszustand der Klimaanlage bzw. der erfindungsgemäßen Luftanreicherungsvorrichtung eine Keimtötung im bestrahlten Luftvolumen stattfindet. Bestrahlungsunterbrechungen können in diesem Fall durch einen Schalter oder eine Zeitschaltuhr, die in den unabhängigen Stromkreis integriert sind, nach Bedarf eingestellt werden. Die UV-Lampe kann direkt auf der erfindungsgemäßen Luftanreicherungs- bzw. Dosiervorrichtung montiert sein und unter anderem auch den Aufnahmeträger 5 bestrahlen.

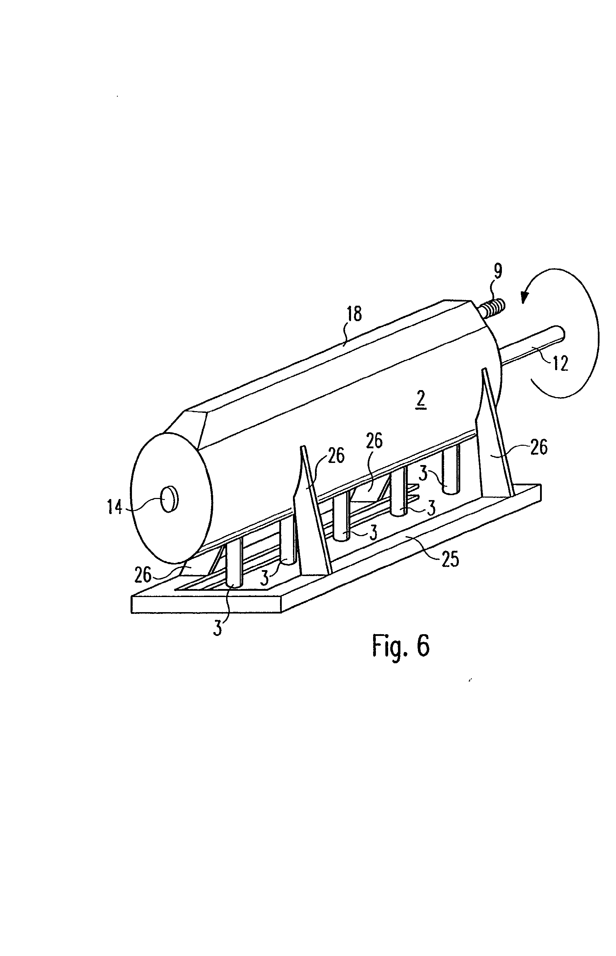

Fig. 6 zeigt das Ausführungsbeispiel in perspektivischer Darstellung. Ein als U-Profil ausgebildeter Rahmen 25 ist über Streben 26 fest mit dem Zylinder 2 verbunden. Der Aufnahmeträger 5 wird in den offenen Bereich des Rahmens 25 eingeschoben und ist nach Montage der Vorrichtung durch das Wandelement 6 des Kanals 1 gesichert. Bei einem Austausch des Aufnahmeträgers 5 wird dieser nach Öffnen des Kanals 1 aus dem Rahmen 25 herausgezogen und durch einen neuen Aufnahmeträger 5 ersetzt. Mittels Klammern oder anderen Halteelementen wirkende Schnellverschlüsse sind ebenfalls denkbar.

Die Fig. 7 und 8 zeigen eine Variante des oben erläuterten Ausführungsbeispiels mit einer Rotationswalze in Form eines Transportrades 113. Das Transportrad 113 ist an einer Welle 112 angebracht und läuft in einem Gehäuse 102, welches mit einem geeigneten Laufraum versehen ist. Das Transportrad 113 ist zu beiden Seiten mit einem radial überstehenden Rand versehen, welcher einen sich um das Transportrad 113 erstreckenden Ringkanal 130 definiert. Der Abstand zwischen dem überstehenden Rand und der Innenwandung des Laufraums ist mit dem Bezugszeichen 131 bezeichnet. An der Unterseite des Gehäuses 102 ist ein Ausflußrohr vorgesehen, dessen Ausflußöffnung oberhalb des Aufnahmeträgers 5 (in Fig. 7 nicht dargestellt) liegt.

Im oberen Bereich des Gehäuses 102 ist ein Dichtungskissen 132 vorhanden, welches in den Ringkanal 130 vorsteht und auf die Umfangsfläche des Transportrades 113 drückt. Das Dichtungskissen 132 weist einen Durchgang auf, an dessen obere Seite die Zuleitung 108 angesetzt ist.

Über die Umfangsfläche des Transportrades 113 sind unter einem Winkel von 72° verteilt 5 Transportvertiefungen 124 vorgesehen. Sobald sich eine Transportvertiefung 124 unterhalb des Durchgangs im Dichtungskissen 132 befindet, wird die Transportvertiefung 124 mit Duftöl gefüllt. Anschließend dreht sich das Transportrad 113 in Pfeilrichtung weiter, und die bemessene Flüssigkeit rinnt innerhalb des Ringkanals 130 nach unten und läuft durch das Ausflußrohr 103 ab.

Das Dichtungskissen 132 muss sowohl beim Befüllen der Transportvertiefung 124 als auch bei jeder anderen Winkelstellung des Transportrades 113 dafür sorgen, dass die Flüssigkeit nicht unkontrolliert in das Gehäuse 102 ausfließt und dieses "überschwemmt". Es hat sich gezeigt, dass ein Silikonkissen der Härte 20 Shore diese Aufgabe zuverlässig und dauerhaft erfüllt.

Die in den Fig. 7 und 8 gezeigte Variante weist den Vorteil auf, dass aufgrund des Abstands 131 außerhalb des Zuleitungsbereichs kein Widerstand zwischen dem Transportrad 113 und der Innenwandung des Gehäuses 102 auftritt.

Zusammenfassend ist festzustellen, dass die erfindungsgemäße Vorrichtung aufgrund ihrer hohen Variabilität für unterschiedlichste Einsatzgebiete geeignet ist und eine stufenlose und exakt einstellbare Beimischung eines Wirkstoffes über einen großen Quantitätsbereich bei gleichmäßiger Geruchsstärke schafft.

## Patentansprüche

1. Vorrichtung zur Luftverbesserung für ein Klimatisierungssystem,
- mit einer Dosiereinrichtung (2, 11, 13; 102, 113) zur steuerbaren Dosierung der pro Zeit abgegebenen Menge eines Wirkstoffes, und
- mit einem in einem Kanal (1) des Klimatisierungssystems vorgesehenen saugfähigen Aufnahmeträger (5), auf welchen der von der Dosiereinrichtung (2, 11, 13; 102, 113) abgegebene Wirkstoff aufdosiert wird.

2. Vorrichtung nach Anspruch 1,
**dadurch gekennzeichnet,**
- **dass** die Dosiereinrichtung (2, 11, 13; 102, 113) mit einer Steuereinheit einer Klimaanlage (17) des Klimatisierungssystems in Verbindung steht.

3. Vorrichtung nach Anspruch 1 oder 2,
**dadurch gekennzeichnet,**
- **dass** der Kanal (1) ein Zentralkanal des Klimatisierungssystems ist.

4. Vorrichtung nach einem der Ansprüche 1 bis 3,
**dadurch gekennzeichnet,**
- **dass** es sich bei der Dosiereinrichtung um eine elektromechanische Dosiereinrichtung (2, 11, 13; 102, 113) handelt.

5. Vorrichtung nach Anspruch 4,
**dadurch gekennzeichnet,**
- **dass** die elektromechanische Dosiereinrichtung (2, 11, 13; 102, 113) einen Elektromotor (11), insbesondere einen Synchron-Elektromotor, als elektromechanischen Antrieb verwendet.

6. Vorrichtung nach Anspruch 5,
**dadurch gekennzeichnet,**
- **dass** die Vorrichtung eine Einheit (15) zur Steuerung der Drehzahl des Elektromotors (11) umfasst.

7. Vorrichtung nach einem der Ansprüche 4 bis 6,
**dadurch gekennzeichnet, dass** die elektromechanische Dosiereinrichtung umfasst:
- ein erstes Teil (2; 102), welches über eine Zuleitung (8; 108) mit einem Wirkstoffreservoir (7) verbunden ist, und welches eine Öffnung zur Abgabe des Wirkstoffes aufweist,
- ein zweites Teil (13; 113), welches eine Transportvertiefung (24.1, 24.2, 24.3, 24.4, 24.5; 124) zur Aufnahme des aus der Öffnung des ersten Teils (13; 113) abgegebenen Wirkstoffes umfasst, und
- einen elektromechanischen Antrieb (11), der eine Relativbewegung der beiden Teile (2, 13; 102, 113) herbeiführt, derart, dass die Transportvertiefung (24.1, 24.2, 24.3, 24.4, 24.5; 124) an der Öffnung zur Abgabe des Wirkstoffes vorbei geführt und dabei befüllt wird.

8. Vorrichtung nach Anspruch 7,
**dadurch gekennzeichnet,**
- **dass** das erste Teil (2; 102) lagefest zu dem Kanal (1) angeordnet ist, und
- **dass** das zweite Teil (13; 113) von dem elektromechanischen Antrieb (11) beaufschlagt wird.

9. Vorrichtung nach Anspruch 7 oder 8,
**dadurch gekennzeichnet,**
- **dass** das erste Teil (2; 102) ein Zylinder ist, dessen Innenwandung mit der Öffnung zur Abgabe des Wirkstoffes versehen ist, und
- **dass** das zweite Teil (13) eine Rotationswalze ist, deren Mantelfläche mit der Transportvertiefung (24.1, 24.2, 24.3, 24.4, 24.5) versehen ist.

10. Vorrichtung nach Anspruch 9,
**dadurch gekennzeichnet,**
- **dass** über den Umfang der Mantelfläche verteilt mehrere Transportvertiefungen (24.1, 24.2, 24.3, 24.4, 24.5; 124; 124) vorgesehen sind.

11. Vorrichtung nach einem der Ansprüche 7 bis 10,
**dadurch gekennzeichnet,**
- **dass** das erste Teil (2) mehrere Öffnungen zur Abgabe des Wirkstoffes und das zweite Teil (13) mehrere Transportvertiefungen (24.1, 24.2, 24.3, 24.4, 24.5) zur Entgegennahme des Wirkstoffes aufweisen.

12. Vorrichtung nach einem der vorhergehenden Ansprüche,
**dadurch gekennzeichnet,**
- **dass** die elektromechanische Dosiereinrichtung (2, 11, 13) mehrere Auslassöffnungen zur Abgabe des Wirkstoffes aufweist.

13. Vorrichtung nach Anspruch 12,
**dadurch gekennzeichnet,**
- **dass** die Auslassöffnungen (3) mittels Verschlüssen (20) selektiv verschließbar sind.

14. Vorrichtung nach einem der vorhergehenden Ansprüche,
**dadurch gekennzeichnet,**
- **dass** der saugfähige Aufnahmeträger (5) aus einem faserhaltigen und/oder offenporigen Material, insbesondere Zellstoff oder einem Saugpapier, besteht.

15. Vorrichtung nach einem der vorhergehenden Ansprüche,
**dadurch gekennzeichnet,**
- **dass** die Vorrichtung eine Halterung (25) für den Aufnahmeträger (5) umfasst, welche baulich fest mit der Dosiereinrichtung (2, 11, 13; 102, 113) verbunden ist.

16. Vorrichtung nach Anspruch 15,
**dadurch gekennzeichnet,**
- **dass** die Halterung (25) als Wechselhalter, insbesondere in Form eines Einschubrahmens, für den Aufnahmeträger (5) ausgebildet ist.

17. Vorrichtung nach einem der vorhergehenden Ansprüche,
**dadurch gekennzeichnet,**
- **dass** der Wirkstoff eine Duftessenz und/oder eine antibakterielle Essenz ist.

18. Vorrichtung nach einem der vorhergehenden Ansprüche,
**dadurch gekennzeichnet,**
- **dass** das Klimatisierungssystem eine Anlage zur Klimatisierung eines Raumes oder eines Gebäudes ist.

19. Vorrichtung nach einem der vorhergehenden Ansprüche,
**dadurch gekennzeichnet,**
- **dass** das Klimatisierungssystem eine Anlage mit mehreren Lüftungskanälen zur Klimatisierung eines Raumes an mehreren Stellen und/oder eines Gebäudes mit mehreren belüfteten Räumen ist, und
- **dass** der Kanal (1) ein Zentralkanal des Klimatisierungssystems ist, von dem die mehreren Lüftungskanäle in Strömungsrichtung hinter der Luftverbesserungsvorrichtung abzweigen.

20. Vorrichtung nach einem der vorhergehenden Ansprüche,
**dadurch gekennzeichnet,**
- **dass** die Dosiereinrichtung (2, 11, 13; 102, 113) zum Einbau in den Kanal (1) des Klimatisierungssystems vorgesehen ist.

21. Vorrichtung nach einem der vorhergehenden Ansprüche,
**dadurch gekennzeichnet,**
- **dass** zusätzlich zur Dosiereinrichtung (2, 11, 13; 102, 113) eine UV-Lampe in dem Kanal (1) vorgesehen ist.

22. Vorrichtung nach einem der vorhergehenden Ansprüche,
**dadurch gekennzeichnet,**
- **dass** die UV-Lampe mit der Dosiereinrichtung (2, 11, 13; 102, 113) elektrisch und/oder mechanisch gekoppelt ist.

23. Verfahren zur Luftverbesserung, mit dem Schritt:
- Dosieren eines Wirkstoffes auf einen saugfähigen Aufnahmeträger (5) in einem Kanal (1) eines Klimatisierungssystems mittels einer steuerbaren Dosiereinrichtung (2, 11, 13; 102, 113).

24. Verfahren nach Anspruch 23, **gekennzeichnet durch** den Schritt:
- Steuern der von der Dosiereinrichtung (2, 11, 13; 102, 113) abgegebenen Dosiermenge des Wirkstoffes in Abhängigkeit von dem Betriebszustand einer Klimaanlage des Klimatisierungssystems.

25. Verfahren nach Anspruch 23 oder 24,
**dadurch gekennzeichnet,**
- **dass** es sich bei der Dosiereinrichtung (2, 11, 13; 102, 113) um eine elektromechanische Dosiereinrichtung handelt, und
- **dass** die Steuerung der Dosiermenge durch das Einstellen der Drehzahl eines Elektromotors (11), insbesondere eines Synchron-Elektromotors, erfolgt, welcher den Antrieb der elektromechanischen Dosiereinrichtung (2, 11, 13; 102, 113) bildet.

26. Verfahren nach Anspruch 25,
**dadurch gekennzeichnet,**
**dass** das elektromechanisch gesteuerte Dosieren die folgenden Schritte umfasst:
- Befüllen einer in einem beweglichen Teil (13) vorhanden Transportvertiefung (24.1, 24.2, 24.3. 24.4, 24.5; 124) mit dem Wirkstoff;
- Verfahren der befüllten Transportvertiefung (24.1, 24.2, 24.3, 24.4, 24.5; 124) zu einer Auslassöffnung (3) der Dosiereinrichtung (2, 11, 13; 102, 113) durch Bewegen des Teils (13; 113) mittels eines elektromechanischen Antriebs (11); und
- Abgeben des Wirkstoffes aus der Transportvertiefung (24.1, 24.2, 24.3, 24.4, 24.5; 124) durch die Auslassöffnung (3) der Dosiereinrichtung (2, 11, 13; 102, 113).

27. Verfahren nach einem der Ansprüche 23 bis 26,
**dadurch gekennzeichnet,**
- **dass** der Wirkstoff eine Duftessenz und/oder eine antibakterielle Essenz ist.
